# EUROPÄISCHE PATENTANMELDUNG

(11) **EP 1 935 423 A2**
(43) Veröffentlichungstag der Anmeldung: **25.06.2008**
(21) Anmeldenummer: 08000547.3
(22) Anmeldetag: 03.09.1998
(51) Int. Cl.: A61K 31/565, A61K 31/57, A61K 45/06

(54) **Zusammensetzung zur Behandlung und/oder Prophylaxe von Tumoren der Brustdrüsen**

(30) Priorität: 11.09.1997 DE 19739916
(62) Teilanmeldung aus: 03002576.1
(71) Anmelder: Wyeth, Madison, NJ 07940 (US)
(72) Erfinder: Hesch, Rolf-Dieter, Prof. Dr., 78464 Konstanz (DE)
(74) Vertreter: Mannion, Sally Kim

(57) **Zusammenfassung**

Verwerdung einer Kombination, aus einem einzelnen·Gestagen, ausgewählt aus der aus Progesteron, Chlormadinonacetat, Norethistronacetat, Cyproteronacetat, Desogestrel, Levonor-gestrel, Antigestagenen, Horinonanalogen mit Gestagen- oder Antigestagenwirkung und Hormonverbindungen, die mindestens ein Gestagen nach Einnahme rasch abspalten, bestehenden Gruppe, und eines einzelnen Estrogens, ausgewahlt aus der aus synthetischem Estrogen, namlich Ethinylestradiol mit einer taglichen Dosis von 1 - 20 µg, Mestranol oder einer Hormonverbindung, die mindestens ein synthetisches Estrogen nach Einnahme rasch abspaltet und biogenen Estragenen, nämlich Estradiol Estriol, Estron, Estran oder einer Hormonverbindung, die mindestens ein biogenes Eströgen nach Einnahme rasch abspaltet bestehen-den Gruppe, als alleinigen hormonellen Wirkstoffen zur Herstellung eines Mittels zur und/oder Prophylaxe von Tumoren der Brustdrüsen durch ununterbrochene orale, transdermale oder Depot-Verabreichung.

## Beschreibung

Die Erfindung betrifft die Verwendung einer Kombination von nur zwei Hormonkomponenten, nämlich eines einzelnen Gestagens und eines einzelnen Estrogens, als alleinigen hormonellen Wirkstoffen, zur Herstellung eines Mittels zur Behandlung und/oder Prophylaxe von Tumoren der Brustdrüsen.

Seit Beginn der Verfügbarkeit hormoneller Kontrazeptiva in den 60er Jahren wurde eine Vielzahl von hormonellen Komponenten hinsichtlich ihrer Eignung in den verschiedensten Darreichungsschemata untersucht. Grundsätzlich ist eine Einteilung in Kombinations- und Sequenzpräparate möglich.

Bei bekannten Kombinationspräparaten wird bespielsweise, sofern die gewünschte Zyklusdauer 28 Tage beträgt, 21 Tage lang in konstanter oder wechselnder absoluter und/oder relativer Dosierung eine Kombination aus einem Estrogenpräparat und einem Gestagenpräparat verabreicht, wobei das Estrogenpräparat z.B. natürliches Estrogen oder synthetisches Ethinylestradiol sein kann, und sich an die Einnahme der vorgenannten 21 Tageseinheiten eine siebentägige Pause anschließt, in der es zu einer die natürliche Monatsblutung simulierenden Entzugsblutung kommt.

Bei den bekannten Sequenzpräparaten wird, wiederum bei einer gewünschten Zyklusdauer von 28 Tage, 7 Tage lang ein reines Estrogenpräparat und dann 15 Tage lang eine Kombination aus einem Estrogenpräparat und einem Gestagenpräparat verabreicht, wobei sich auch hier wieder eine einnahmefreie Zeit von z.B. 6 Tagen anschließt, in der es zur Entzugsblutung kommt. Es ist zwar bereits bekannt, die den Kombinations- und dem Sequenzpräparaten eigenen Einnahmepausen im Interessen einer größeren Einnahmesicherheit dadurch zu überbrükken, daß innerhalb der betreffenden Tage Placebos verabreicht werden, jedoch ist man bisher stets davon ausgegangen, daß während der etwa einwöchigen Einnahmepause keine Hormone der hier in Rede stehenden Art verabreicht werden dürfen, um eine zuverlässige Entzugsblutung zu gewährleisten. Lediglich bei Substitutionspräparaten in der Menopause der älteren Frauen hat man während des Gesamtzyklus Hormone verabreicht, beispielsweise in der Abfolge 10 Tage Estrogenpräparat, 11 Tage Kombination aus Estrogen- und Gestagenpräparat, 7 Estrogenpräparat, 7 Estrogenpräparat in besonders niedriger Dosierung, jedoch sind diese Substitutionspräparate zur Ovulationshemmung nicht geeignet.

Die bei Substitutionstherapie verwendeten Sequenzpräparate sind zur Kontrazeption insbesondere deshalb ungeeignet, weil das natürliche Estradiol in der gegebenen Dosierung die Ovulation nicht verhindert und die Phase, in der Gestagen verabreicht wird, mit nur 11 Tagen zu kurz ist. Die vorstehend beschriebene sequentielle Anordnung gewährleistet bei den Substitutionspräparaten jedoch eine relativ gute Zykluskontrolle.

In der EP 0 309 263 A1 ist ein Kontrazeptionsmittel beschrieben, welches im wesentlichen aus Blöcken von Estrogen-Gestagen-Kombinationen besteht, wobei jeder Block entweder Estrogen- oder Gestagen-Dominanz aufweist, und während 21 - 24 Tagen des 28-Tage-Zyklus der Frau eingenommen wird. An die vorgenannte Hormon-Verabreichungsphase von 21 - 24 Tagen schließt sich zwingend eine 4 - 7 Tage umfassende hormonfreie Einnahmepause an, wobei während dieser hormonfreien Tage beispielsweise ein Placebo verabreicht werden kann. Durch die zwingend vorgeschriebene Einnahmepause wird eine Abbruchblutung bewirkt, die bei der Vorgehensweise nach der EP 0 309 263 A1 als zwingend notwendig angesehen wird.

Aus der EP 0 628 312 A1 ist ein Kombinationspräparat zur Kontrazeption bekannt, welches aus einer oder mehreren Stufen besteht. Dabei ist vorgesehen, daß mindestens eine Stufe die Kombination von drei Komponenten, nämlich einem biogenen Estrogen, einem synthetischen Estrogen und einem Gestagen enthält und die weiteren Stufen jeweils aus einem pharmazeutisch unbedenklichen Placebo oder einem biogenen oder synthetischen Gestagen, oder einem biogenen oder synthetischen Estrogen, oder einer Kombination aus zwei Komponenten, nämlich einem biogenen Estrogen, einem synthetischen Estrogen und einem Gestagen oder einer Kombination aus synthetischen Estrogen und einem Gestagen, bestehen. Mit dem darin beschriebenen Stufenkonzept erfolgt typischerweise eine Zustandsänderung über die Zeit. Eine derartige Zustandsänderung kann sowohl dergestalt erfolgen, daß sich die Zusammensetzung der die Stufe ausbildenden Phasen hinsichtlich der eingesetzten Komponenten verändert, als auch dadurch, daß sich lediglich die Konzentrationen der in den die Stufe ausbildenden Phasen eingesetzten Komponenten ändern. Aus der Gesamtoffenbarung der vorgenannten Druckschrift, einschließlich sämtlicher Beispiele, geht hervor, daß dort von der bis dahin als unerläßlich angesehenen Praxis nicht abgegangen werden soll, durch Gestagenentzug eine Abbruchblutung zu bewirken.

Auch bei einer aus der DE-OS 44 05 898 bekannten Vorgehensweise wird die Unterdrückung des Menstruationszyklus nicht in Erwägung gezogen. Die Verwendung eines Estrogens und eines Gestagens in transdermaler Applikation zur Kontrazeption wird zwar diskutiert, jedoch werden in diesem Zusammenhang ausdrücklich die Zyklusregulierung und die Zyklusstabilisierung angesprochen.

Schließlich ist in der nicht vorveröffentlichten, aber prioritätsälteren EP 0 911 029 A2 eine Kontrazeptionsbehandlung beschrieben, bei der eine Kombination aus Estrogen und Gestagen, nämlich etwa 5 - 35 µg Ethinylestradiol und etwa 0,025 bis 10 mg Norethindronacetat, für bis zu 110 aufeinander folgender Tage monophasisch verabreicht werden, jedoch ist auch hier zwingend eine daran anschließende Einnahmepause von 3 bis 10 Tagen vorgeschrieben, um eine Abbruchblutung hervorzurufen, ehe dann ein neuer Hormon-Einnahmezyklus beginnt.

Aus Contraception 51, Seiten 355 - 358, veröffentlicht im Jahr 1995, ist ferner eine Versuchsbeschreibung bekannt, bei der einer Gruppe von Patientinnen über einen Zeitraum von einem Jahr jeweils für 21 Tage eine tägliche Dosis von 0,25 mg Levonorgestrel und 0,05 mg Ethinylestradiol, also eine sehr hohe Dosis, gefolgt von einer siebentägigen Einnahmepause, verabreicht wurden, während eine Vergleichsgruppe für den gesamten Jahreszeitraum die vorgenannte Hormonkombination täglich ohne Einnahmeunterbrechung erhielt, resultierend in einer deutlich höheren Hormonverabreichung auf Monatsbasis bei der letztgenannten kontinuierlichen Vergleichsgruppe im Verhältnis zu der erstgenannten. Die Hormonverabreichung erfolgte bei beiden Gruppen ausschließlich vaginal, wobei hinsichtlich einer zusätzlichen Kontrollgruppe, bei der die Verabreichung oral erfolgte, ausdrücklich festgestellt ist, daß sich hierbei im Vergleich zur vaginalen Verabreichung keine Unterschiede zeigten. Die Untersuchung ergab, daß bei der kontinuierlichen Verabreichung der vorgenannten Hormonkomponenten in erheblichem Maße Zwischenblutungen auftraten, so daß die Akzeptanz der dort beschriebenen Vorgehensweise vor allem bei Frauen des westlichen Kulturkreises - die in der vorgenannten Veröffentlichung diskutierten Untersuchungen wurden in einer Drittweltumgebung vorgenommen - nicht gegeben ist und die vorgenannte Versuchsbeschreibung eher Anlaß geben könnte, von einer kontinuierlichen Verabreichung der beschriebenen Hormonkombination abzusehen und stattdessen monatliche Zwangs-Einnahmepausen vorzusehen.

Aus einem Artikel in American Journal of Hematology 52, S. 237/238, veröffentlicht im Jahr 1996, ist ein Vorschlag bekannt, für drei Monate ein orales Kontrazeptionsmittel, welches aus 150 mg Levonorgestrel und 30 µg Ethinylestradiol, also auch hier eine recht hohe Ethinylestradioldosis, zu verabreichen, wobei dies zur Behandlung einer Patientin mit kongenitaler Afibrinogenemie diente, um auf diese Weise sowohl eine Menstruation zu verhindern als auch eine Ovulation zu unterdrücken. In dem Artikel heißt es, daß die vorgeschriebene Kontrazeptionsbehandlung besser kontinuierlich erfolgen soll, um bei Patientinnen mit kongenitaler Afibrinogenemie und dem daraus resultierenden Blutverlust infolge von Menstruation eine Amenorrhö. Es handelt sich hierbei um die Verwendung eines Kontrazeptionsmittels zur Behandlung einer spezifischen Erkrankung, für die exzessive Menstruationsblutungen typisch sind, wobei aber selbst zu diesem Zweck keine kontinuierliche Hormonverabreichung für einen längeren Zeitraum als drei Monate empfohlen wird.

Insgesamt ist festzustellen, daß es bis zum Vorschlag gemäß der nicht vorveröffentlichten DE-OS 197 05 229, bei der allerdings zwingend die Verwendung von drei Hormonkomponenten zur hormonalen Kontrazeption zur Behandlung und/oder Prophylaxe von Tumoren der Brustdrüsen vorgesehen ist, als unerläßlich angesehen wurde, eine Abbruchblutung herbeizuführen, während eine kontinuierliche Verabreichung von zwei Komponenten, nämlich einem Estrogen und einem Gestagen, wie z.B. in der DE-PS 44 05 591 beschrieben, lediglich als zur postmenopausalen Hormonsubstitution geeignet angesehen wurde.

Der Erfindung liegt die Aufgabe zugrunde, ein Mittel zur Behandlung und/oder Prophylaxe von Tumoren der Brustdrüsen bereitzustellen, mittels dessen, unter gleichzeitiger Erzielung einer zuverlässigen hormonalen Kontrazeptionswirkung, Regelblutungen ausgeschlossen und gewährleistet wird, daß Zwischenblutungen in wesentlich geringerem Maße auftreten als bei vaginaler Applikation, wobei das Mittel auf anderem Wege als vaginal sowie als langdauernde Medikation verabreicht werden soll. Weiterhin sollen die ansonsten bei hormonalen Mitteln zur Kontrazeption beobachteten Nebenwirkungen weiter reduziert werden.

Erfindungsgemäß wird diese Aufgabe durch die Merkmalskombination des Patentanspruches 1 gelöst.

Besonders bevorzugte Ausführungsformen der Erfindung sind Gegenstand der Unteransprüche.

Wird ein biogenes Estrogen verwendet, so kann die Tagesdosis z. B. im Falle von Estradiol 0,1 - 2mg betragen.

Unter Estrogenen sollen gemäß der heutigen Auffassung hierin Steroidmoleküle verstanden sein, die ihre Wirkung vorzugsweise dadurch entfalten, daß sie an verschiedenen Zellorten in verschiedenen Organen in unterschiedlicher Weise biologische Wirkung ausüben. Estrogene können wirken an (1) der Zellmembran, (2) intrazellulären zytoplasmatischen Proteinen und (3) an spezifischen Zellkemrezeptoren. In jüngster Zeit ist bekannt geworden, daß neben dem klassischen Estrogenrezeptortyp 1 ein zweiter Estrogenrezeptortyp 2 beschrieben wurde, dessen Organverteilung anders ist als die des Estrogenrezeptortyp 1.

Die vorgenannte Definition umfaßt somit auch jene als "Designerhormone" bezeichneten Verbindungen, die die vorgenannten Eigenschaften aufweisen.

Biogene Estrogene sind demzufolge Steroidmoleküle, die eine estrogenartige Wirkung an der Membran, an zytoplasmatischen Proteinen und an Kernrezeptoren für hydrophobe Ringsubstanzen entfalten und dadurch biologische Wirkungen auslösen, die einer hydrophoben Steroidringstruktur entsprechen, die estrogenartige Wirkung in Zellen, Organen und dem Gesamtorganismus auszulösen in der Lage sind.

Unter biogenen Estrogenen sollen darüber hinaus auch solche Estrogene verstanden werden, die vom menschlichen Körper erzeugt werden und umfassen somit körpereigene Estrogene. Die in bestimmten Ausführungsformen des erfindungsgemäßen Mittels verwendeten biogenen Estrogene sind typischerweise solche, die chemisch synthetisiert werden. Grundsätzlich ist jedoch auch die. Verwendung derartiger, aus einem Organismus isolierter Verbindungen möglich.

Desweiteren sollen unter biogenen Estrogenen hierin auch konjugierte biogene Estrogene wie beispielsweise Estradiolvalerat und Estronsulfat verstanden werden.

Unter Antiestrogenen sollen hierin hydrophobe Ringstruktursubstanzen und andere Substanzen, die der vorbeschriebenen Estrogenwirkung wiederum an Zellen, Organen oder im Gesamtorganismus spezifisch und selektiv entgegenwirken können, verstanden werden.

Unter kontinuierlicher Verabreichung wird hierin eine über den Anwendungszeitraum ununterbrochene Verabreichung verstanden, bei der keine bezüglich der Hormonkomponenten einnahmefreien Intervalle vorgesehen sind. Dies bedeutet auch, daß eine Unterbrechung der Verabreichung des Mittels durch Gabe von Placebos anstelle des hormonalen Mittels nicht vorgesehen ist In der Folge kommt es über die gesamte, typischerweise die Zeitspanne mehrerer Monate bis Jahre umfassenden Verabreichungsdauer zu keinen Veränderungen der grundsätzlichen Zusammensetzung der Hormonkomponenten. Vielmehr werden über die gesamte Verabreichungsdauer ununterbrochen und unverändert die das erfindungsgemäße hormonale Mittel ausbildenden Hormonkomponenten in unveränderter Konzentration verabreicht. Denkbar ist jedoch, daß die Konzentration von Estrogen, verstanden in der gesamten hierin definierten Breite des Begriffs, und Gestagen, ebenfalls verstanden in der gesamten hierin definierten Breite des Begriffs, bei älteren Frauen gegenüber jüngeren verändert wird. Dies kann auch so geschehen, daß entlang der kontinuierlichen Applikation zunächst mit einer bestimmten Zusammensetzung begonnen wird und diese dann im Laufe von Wochen, Monaten und Jahren an die geänderten biologischen Bedürfnisse der Frau durch die Applikation eines Folgepräparates angepaßt wird, das jedoch auch ein Mittel gemäß der vorliegenden Erfindung umfaßt.

Infolge der kontinuierlichen Applikation der genannten Hormonkomponenten wird gewährleistet, daß die natürlicherweise im weiblichen Organismus ablaufenden hormonalen Vorgänge die kontrazeptive Sicherheit nicht durchbrechen.

Durch die Estrogen-Komponerite respektive durch spezifische Wirkung Von hydrophoben Ringsubstanzen mit estrogenartiger Wirkung kann es zu einer Suppression der Gonadoprotine kommen. Dies ist erwünscht. Die daraus resultierende Unterdrückung der Ovarialfunktion wird ausgeglichen durch eine adäquate Substitution von Estrogenwirkung. Damit kommt es zu einer Verhütung der Entwicklung einer Osteoporose, die günstigen Gefäßwirkungen von Estrogenen bleiben erhalten, ebenso wird der Lipidostoffwechsel nicht ungünstige beeinflußt. Durch Unterbrechung der zyklusabhängigen Instabilität im Hormonsystem kann das prämenstruelle Syndrom günstig beeinflußt werden, darüber hinaus wird die Homöostase des Gerinnungssystems nicht gestört, da das labile Gleichgewicht, in welchem sich das Gerinnungssystem befindet, nicht durch das Auf und Ab der Hormonschwankungen,aktiviert und desaktiviert wird. Deshalb eignet sich das hormonale Mittel nach der Erfindung insbesondere auch für Frauen im Alter von mehr als 40 Jahren, bei denen die Gefahr von Durchblutungsstörungen mit steigendem. Alter bekanntlich zunimmt. Ebenso wird die Thrombosegefahr, die in jüngster Zeit eine erhebliche Bedeutung bei der kontrazeptiven Therapie erlangt hat, vermindert.

Überraschenderweise wurde auch festgestellt, daß bei der Verabreichung des erfindungsgemäßen Mittels zuverlässig eine kontinuierliche Unterdrückung des Menstruationszyklus und der Monatsblutung bei ausgesprochen niedriger Dosierung möglich ist. Ohne im folgenden darauf festgelegt werden zu wollen, scheint die Kombination der genannten beiden Hormonkomponenten, und insbesondere die geringe Dosierung der Estrogene darin, geeignet, die sonst üblichen Nebenwirkungen des Ethinylestradiols zu beseitigen und die ansonsten bei Kontrazeptiva nach dem Stand der Technik typischerweise erforderlichen Gaben von mehr als 15 µg Ethinylestradiol zu unterschreiten.

Die niedrige Dosierung der beiden Hormonkomponenten, und insbesondere der Estrogenkomponente, wird durch die additive Wirkung der beiden Hormonkomponenten ermöglicht, ohne daß es zu einer Beeinträchtigung der Wirkung des erfindungsgemäßen Mittels hinsichtlich seiner kontrazeptiven sowie ovulationshemmenden Eingenschaften kommt.

Die durch das erfindungsgemäße Mittel zuverlässig gewährleistete Ovulationshemmung und Unterdrückung des Menstruationszyklus ist für bestimmte Patientinnen von großer Bedeutung, wie z.B. für Spitzensportlerinnen, Tänzerinnen, und Geschäftsfrauen, die die Beeinträchtigung ihrer körperlichen, geistigen sowie emotionalen Leistungsfähigkeit durch den Menstruationszyklus ausschließen wollen. Infolge der kombinierten und kontinuierlichen Verabreichung der beiden Hormonkomponenten des erfindungsgemäßen Mittels ist es möglich, dieses entweder oral, transdermal, durch Depot-Injektionen oder Hormonimplantate, zu verabreichen. Dabei können auch im vorliegenden Fall die für die jeweiligen Applikätionsformen beobachteten Vorteile realisiert werden.

Als orale Darreichungsformen sind all im Stand der Technik bekannten Formen wie beispielsweise Tabletten, Dragees, Pillen oder Kapseln möglich, die unter Verwendung der üblichen Hilfs-und Trägerstoffe hergestellt werden. ,

Bei der transdermalen Verabreichung des erfindungsgemäßen Mittels können die beiden das Mittel ausbildenden Hormonkomponenten beispielsweise auf ein Pflaster aufgebracht werden oder auch mittels transdermaler therapeutischer Systeme appliziert und somit dem Organismus zugeführt werden, wobei beispielsweise eine bereits vorgefertigte Kombination der beiden Hormonkomponenten oder diese einzeln in ein derartiges System eingebracht werden, welches auf Iontophorese oder Diffusion oder ggf. einer Kombination dieser Effekte beruht.

Für den Fall der oralen Applikation hat es sich als sinnvoll erwiesen, daß Tageseinheiten, die jeweils eine Kombination der beiden Hormonkomponenten umfassen, räumlich getrennt und einzeln entnehmbar in einer Verpackungseinheit angeordnet sind, so daß ein leichte Kontrolle möglich ist, ob tatsächlich die typischerweise tageweise einzunehmende orale Zubereitungsform bereits eingenommen wurde oder nicht. Wichtig ist dabei, daß gewährleistet wird, daß keine einnahmefreien Tage auftreten können. Depot-Injektionen können im Abstand von 1 bis 6 Monaten oder noch länger appliziert werden; Hormonimplantate enthalten die beiden Hormonkomponenten und geben diese über die Dauer von vorzugsweise 3 bis 6 Monaten ab.

Bei der Anwendung des erfindungsgemäßen Mittels hat sich überraschend gezeigt, daß die Behandlung und/oder Prophylaxe von Tumoren der Brustdrüsen möglich ist. Es ist eine Erkenntnis der jüngsten Risikoforschung für Brustkrebs, daß dieser sich dadurch einstellt, daß in bestimmten Risikogenen Mutationen auftreten, die angeboren oder erworben sein können. Die moderne Krebstherapie geht davon aus, daß auf einem der beiden Allele eines Gens eine krebsauslösende Mutation vorhanden ist, die zunächst von dem anderen gesunden Allel noch kontrolliert wird. Kommt es auch auf dem zweiten Allel im Laufe des Lebens zu einer weiteren Mutation in einer bestimmten Organzelle, so kann diese zum unkontrollierten, bösartigen Wachstum überführt werden.

Mutationen auf dem zweiten Allel treten besonders häufig zu bestimmten Phasen des Zellzyklus auf, nämlich in der sogenannten G1-Phase. Der Menstruationszyklus treibt die Brustzelle alle vier Wochen in einen Zellzyklus, "öffnet" das Genom für Mutationen, die entweder repariert oder apoptotisch "abgeräumt" werden. Unter den Bedingungen der klassischen kombinierten oder sequentiellen Kontrazeptionsbehandlung kann eine Frau 500 bis 700 Zyklen im Laufe ihres Lebens haben, während unter Naturbedingungen eine Frau maximal 20 bis 30 Zyklen hat. Damit wird in einer ungewöhnlich häufigen Anzahl von Zellzyklen über jeweils 8 Tage hinweg ein erhebliches Mutationsrisiko in das stimulierte Brustdrüsengewebe hineingetragen. Unterdrückt man den Menstruationszyklus, wie dies mit dem erfindungsgemäßen Mittel möglich ist, so bringt man die Brustzellen in eine "Ruhephase" und es ist wissenschaftlich gesichert, daß in der Ruhephase weniger Krebs auslösende Mutationen in ein Gewebe hineinkommen, als in einem stimulierten Gewebe. Dadurch wird die Mutagenese, d.h. das Brustkrebsrisiko, um ein Vielfaches verringert.

Die vorgenannte Verwendung des erfindungsgemäßen Mittels zur Behandlung und/oder Propylaxe von Tumoren der Brustdrüsen ist insbesondere dann mit ganz besonderen Vorteilen verbunden, wenn es sich bei den Anwenderinnen des Mittels um Risikoträgerinnen, wie beispielsweise solche mit familiären Brustdrüsenkrebsrisiko, handelt.

Die Menge der verabreichten Gestagene und Estrogene, soweit nicht in Patentanspruch 1 definiert, entspricht im wesentlichen der Menge vergleichbarer Präparate nach dem Stand der Technik. Weitere Angeben betreffend die - täglich - zu verabreichenden Mengen verschiedener, die erste bzw. zweite Hormonkomponente ausbildender Verbindungen finden sich in den Beispielen.

### Beispiel 1:

Zur Behandlung eines Tumors der Brustdrüsen wurde ein Mittel verwendet, welches pro in Tablettenform vorliegender Tageseinheit 5 µg Ethinylestradiol und 2 mg enthielt. Norethisteronacetat Dabei ist beachtlich, daß Norethisteronacetat in einem Konzentrationsbereich von 0,5 - 5 mg zur Anwendung gelangen kann. Das Mittel wurde über 9 Monate verabreicht und zeigte eine sehr gute kontrazeptive Sicherheit unter vollständiger Unterdrückung des Menstruationszyklus und praktisch keine Nebenwirkungen. Im Rahmen der hier vorgestellten Untersuchung wurde sichergestellt, daß die Probandinnen das Mittel täglich, d.h. ohne Einnahmepause, über den gesamten obengenannten Zeitraum einnahmen.

### Beispiel 2:

Zur Behandlung eines Tumors der Brustdrüsen wurde ein Mittel verwendet, welches pro in Tablettenform vorliegender Tageseinheit 0,5 mg Estriol und 2 mg Chlormadinonacetat enthielt. Dabei ist beachtlich, daß Estriol in einem Konzentrationsbereich von 0,5 bis 3 mg und Chlormadinonacetat in einem Konzentrationsbereich von 0,75 - 5 mg zur Anwendung gelangen kann. Das Mittel wurde ohne Einnahmepause über 12 Monate verabreicht. Die Wirkungsweise entsprach derjenigen von Beispiel 1.

### Beispiel 3:

Zur kontrazeptiven Behandlung wurde ein Mittel verwendet, welches pro in Tablettenform vorliegender Tageseinheit 0,5 mg Estradiolvalerat und 2 mg Lynestrenol enthielt. Dabei ist beachtlich, daß Estradiolvalerat in einem Konzentrationsbereich von 0,5 - 5 mg und Lynestrenol in einem Konzentrationsbereich von 0,5-4,5 mg zur Anwendung gelangen kann. Das Mittel wurde ohne Einnahmepause über 12 Monate verabreicht. Die Wirkungsweise entsprach derjenigen von Beispiel 1.

### Beispiel 4:

Zur kontrazeptiven Behandlung wurde ein Mittel verwendet, welches pro in Tablettenform vorliegender Tageseinheit 7,5 µ Ethinylestradiol und 75 µg Desogestrel enthielt. Dabei ist beachtlich, daß Desogestrel in einem Konzentrationsbereich von 50 - 200 µg zur Anwendung gelangen kann. Das Mittel wurde ohne Einnahmepause über 12 Monate verabreicht. Die Wirkungsweise entsprach derjenigen von Beispiel 1.

### Beispiel 5:

Zur kontrazeptiven Behandlung wurde ein Mittel verwendet, welches pro in Tablettenform vorliegender Tageseinheit 20 mg Tamoxifen und 2 mg Lutenyl enthielt. Dabei ist beachtlich, daß Tamoxifen in einem Konzentrationsbereich von 10 - 50 mg und Lutenyl in einem Konzentrationsbereich von 1 - 5 mg zur Anwendung gelangen kann. Dieses Mittel ist vorzugsweise geeignet für eine Kontrazeption bei Frauen mit familiärem Brustdrüsenkrebsrisiko. Das Mittel wurde ohne Einnahmepause über 12 Monate verabreicht, die Wirkungsweise entsprach derjenigen von Beispiel 1.

### Beispiel 6:

Zur kontrazeptiven Behandlung wurde ein Mittel verwendet, welches pro in Tablettenform vorliegender Tageseinheit 50 mg Raloxifen und 2,5 mg Medroxyprogesteronacetat (MPA) enthielt. Dabei ist beachtlich, daß Raloxifen in einem Konzentrationsbereich von 30 - 100 mg und Medroxyprogesteronacetat in einem Konzentrationsbereich von 2 - 10 mg zur Anwendung gelangen kann. Diese Kombination eignet sich vorzugsweise bei Frauen mit familiärem Brustdrüsenkrebsrisiko und jungen Frauen nach durchgemachten Brustdrüsenkrebs. Das Mittel wurde ohne Einnahmepause über 12 Monate verabreicht, die Wirkungsweise entsprach derjenigen von Beispiel 1.

### Beispiel 7:

Zur kontrazeptiven Behandlung wurde ein Mittel verwendeet, welches pro in Tablettenform vorliegender Tageseinheit 10 µg Ethinylestradiol und Tibolon in einer Konzentration von 2mg täglich enthält. Dabei ist beachtlich, daß Tibolon in einer Konzentration von 1 - 10 mg zur Anwendung gelangen kann. Das Mittel wurde ohne Einnahmepause über 12 Monate verabreicht. Die Wirkungsweise entsprach derjenigen von Beispiel 1.

### Beispiel 8:

Zur kontrazeptiven Behandlung wurde ein Mittel verwendet, welches pro in Tablettenform vorliegender Tageseinheit 10 µg Ethinylestradiol enthält und als Antigestagen die Substanz Ro486 in einer Konzentration von 2,5 mg. Dabei ist beachtlich, daß Ro486 in einem Konzentrationsbereich von 1 - 7,5 mg zur Anwendung gelangen kann. Das Mittel wurde ohne Einnahmepause über 12 Monate verabreicht, die Wirkungsweise entspricht derjenigen von Beispiel 1.

Die in der vorstehenden Beschreibung und in den Ansprüchen offenbarten Merkmale der Erfindung können sowohl einzeln als auch in beliebiger Kombination für die Verwirklichung der Erfindung in ihren verschiedenen Ausführungsformen wesentlich sein.

## Patentansprüche

1. Verwendung einer Kombination aus einem einzelnen Gestagen, ausgewählt aus der aus Progesteron, Chlormadinonacetat, Norethisteronacetat, Gyproteronacetat, Desogestrel, Levonorgestrel, Antigestagenen, Hormonanalogen mit Gestagen- oder Antigestagenwirkung und Hormonverbindungen, die mindestens ein Gestagen nach Einnahme rasch abspaltek bestehenden Gruppe, und eines einzelnen Estrogens, ausgewählt aus der aus synthetischem Estrogen, nämlich Ethinylestradiol mit einer täglichen Dosis von 1 - 20 µg, Mestranol oder einer Hormonverbindung, die mindestens ein synthetisches Estrogen nach Einnahme rasch abspaltet und biogenen Estragenen, nämlich Estradiol, Estriol, Estron, Estran oder einer Hormonverbindung, die mindestens ein biogenes Estrogen nach Einnahme rasch abspaltet, bestehenden Gruppe, als alleinigen hormonellen Wirkstoffen zur Herstellung eines Mittels zur und/oder Prophylaxe von Tumoren der Brustdrüsen durch ununterbrochene orale, transdermale oder Depot-Verabreichung.

2. Verwendung nach Anspruch 1, **dadurch gekennzeichnet, daß** als Estrogen Ethinylestradiol in einer täglichen Dosis von 5 -10 µg verwendet wird.

3. Verwendung nach Anspruch 1. oder 2, **dadurch gekennzeichnet, daß** Levonorgestrel und Ethinylestradiol als alleinige hormonelle Wirkstoffe verwendet werden.

4. Verwendung nach Anspruch 3, **dadurch gekennzeichnet, daß** das Mittel zur oralen Verabreichung bestimmt ist.

5. Verwendung nach Anspruch 3 oder 4, **dadurch gekennzeichnet, daß** der bestimmungsgemäße Verabreichungszeitmum wenigstens neun Monate beträgt.

6. Verwendung nach Anspruch 5, **dadurch gekennzeichnet, daß** der bestimmungsgemäße Verabreichungszeitraum wenigstens zwölf Monate beträgt.
